# EUROPÄISCHE PATENTSCHRIFT

(11) **EP 1 096 997 B1**
(45) Veröffentlichungstag und Bekanntmachung des Hinweises auf die Patenterteilung: **29.12.2004**
(21) Anmeldenummer: 99931210.1
(22) Anmeldetag: 29.06.1999
(51) Int. Cl.: B01J 21/16, B01J 37/10, C07C 29/04, B01J 37/06

(54) **ENTALUMINIERTER KATALYSATORTRÄGER, VERFAHREN ZUR VERRINGERUNG DES ALUMINIUMGEHALTS EINES KATALYSATORTRÄGERS UND VERFAHREN ZUR HYDRATISIERUNG VON C2- ODER C3-OLEFINEN MIT WASSER IN GEGENWART EINES KATALYSATORS, DER AUS DIESEM MIT SÄURE GETRÄNKTEM KATALYSATORTRÄGER BESTEHT**
DEALUMINIZED CATALYST CARRIER, METHOD FOR DECREASING THE ALUMINIUM CONTENT OF SAID CATALYST CARRIER AND METHOD FOR HYDRATING C2- OR C3- OLEFINS WITH WATER IN THE PRESENCE OF A CATALYST CONSISTING OF AN ACID-SATURATED CATALYST CARRIER
SUPPORT DE CATALYSEUR DESALUMINE, PROCEDE PERMETTANT DE DEMINUER LE CONTENU D'ALUMINE DUDIT SUPPORT DE CATALYSEUR ET PROCEDE D'HYDRATATION D'OLEFINES C2 OU C3 AVEC DE L'EAU EN PRESENCE D'UN CATALYSEUR COMPOSE DUDIT SUPPORT DE CATALYSEUR IMPREGNE D'ACIDE

(30) Priorität: 03.07.1998 DE 19829747
(43) Veröffentlichungstag der Anmeldung: 09.05.2001
(73) Patentinhaber: Degussa AG, 40474 Düsseldorf (DE); RWE-DEA Aktiengesellschaft für Mineraloel und Chemie, 22297 Hamburg (DE)
(72) Erfinder: SAKUTH, Michael, D-45772 Marl (DE); MASCHMEYER, Dietrich, D-45657 Recklinghausen (DE); LOHRENGEL, Gregor, D-46286 Dorsten (DE); STOCHNIOL, Guido, D-45657 Marl (DE)
(86) Internationale Anmeldenummer: PCT/EP1999/004486
(87) Internationale Veröffentlichungsnummer: WO 2000/001481

(56) Entgegenhaltungen:
- EP-A- 0 283 649
- EP-A- 0 503 229
- DE-B- 1 156 772
- GB-A- 1 306 141

## Beschreibung

Beansprucht wird ein entaluminierter Katalysatorträger, ein Verfahren zur Herstellung des Katalysatorträgers und ein Verfahren zur Hydratisierung von C₂- oder C₃-Olefinen mit Wasser in Gegenwart eines Katalysators, der aus diesem mit Säure getränkten Katalysatorträger besteht.

Bekannt ist, daß sich lineare oder gering verzweigte Olefine niederer Molekülmasse in der Gasphase bei erhöhten Drücken und Temperaturen mit Wasserdampf zu Alkoholen umsetzen lassen. Von großtechnischer Bedeutung ist dabei die Synthese von Ethanol aus Ethen und Isopropanol aus Propen. Die Herstellung dieser Alkohole erfolgt in Gegenwart saurer Katalysatoren, wobei in der Regel ein mit Phosphorsäure getränktes, alumosilikatisches bzw. silikatisches Material als Katalysatorträger verwendet wird.

Das Material des Katälysatorträgers baut sich entweder aus reiner Kieselsäure auf, wie beispielsweise Kieselgel (US 2 579 601), oder besteht aus Kieselsäure mit variierenden Tonerdegehalten (US 3 311 568) bzw. aus reinen, beispielsweise montmorillonithaltigen Schichtsilikaten (DE 29 08 491).

Neben diesen phoshorsäurehaltigen Katalysatorträgern werden auch zeolithische Materialien (EP 0 323 269 B1) oder andere saure Katalysatoren, wie z.B. Zirkonphosphate (GB 005 534), verwendet.

Bei den Trägern, die ausschließlich auf Kieselsäure in Form von Kieselgelen basieren, ist die mechanische Festigkeit über eine längere Standzeit bisher problematisch. Aluminiumhaltige Katalysatorträger oder solche, die nur auf Tonerden basieren, zeigen zwar eine deutlich höhere Langzeitstabilität, besitzen aber den erheblichen Nachteil, daß während der Hydratisierungsreaktion Aluminium durch die Einwirkung der Phosphorsäure aus dem Katalysatorträger herausgelöst wird. Das Aluminium findet sich als schwerlösliche Ablagerung in Form von Aluminiumphosphaten in den nachgeschalteten Apparaten wieder. Diese werden hierdurch allmählich verblockt.

In DE 1 156 772 wird ein Verfahren vorgestellt, den Aluminiumgehalt der Schichtsilikate durch die Einwirkung von Salzsäure zu senken. Jedoch weist das Trägermaterial selbst bei intensiver Salzsäurewäsche noch einen Restaluminiumgehalt von etwa 1 bis 2 Gew.-% auf.

In EP 0 578 441 B1 wird mit einem pelletisierten Silikatträger auf Aerosilbasis (Degussa), der kein Aluminium enthält, eine gewisse Langzeitstabilität erreicht. Ausgangsmaterial für die Herstellung von Aerosil ist das relativ teure Siliziumtetrachlorid. Da es sich bei Materialien auf Basis von Schichtsilikaten, wie beispielsweise dem Montmorillonit, um ein Naturprodukt handelt, das in entsprechenden Lagerstätten abgebaut werden kann, besitzen diese gegenüber pelletisierten Silikatträgern einen deutlichen Vorteil in der Wirtschaftlichkeit des Hydratisierungsprozesses.

Der vorliegenden Erfindung liegt daher die Aufgabe zugrunde, ein wirtschaftliches Verfahren zur Hydratisierung von C₂- oder C₃-Olefinen mit Wasser in Gegenwart eines Katalysators, der aus einem mit Säure getränkten Katalysatorträger besteht, zu finden, bei welchem der Katalysatorträger eine möglichst hohe Langzeitstabilität besitzt und gleichzeitig der Austrag an Aluminium während der Hydratisierungsreaktion möglichst gering ist.

Überraschenderweise wurde gefunden, daß ein entaluminierter Katalysatorträger, auf Basis von im wesentlichen aluminiumhaltigen Schichtsilikaten mit Montmorillonit-Struktur, mit einem Aluminiumgehalt kleiner 0,3 Gew.-% eine hohe Langzeitstabilität aufweist und daß bei einem Verfahren zur Hydratisierung von C₂- oder C₃-Olefinen mit Wasser in Gegenwart eines Katalysators, der aus einem mit Säure getränkten Katalysatorträger mit einem Aluminiumgehalt nach der Behandlung von Kleiner 0.3 gew. % besteht, durch Durchführen der Hydratisierungsreaktion mit einem entaluminierten Katalysatorträger gemäß zumindest einem der Ansprüche 1 bis 24, keine oder nur geringe Mengen Aluminium aus dem Katalysatorträger ausgewaschen werden.

Gegenstand der vorliegenden Erfindung ist deshalb ein entaluminierter Katalysatorträger, auf Basis von im wesentlichen aluminiumhaltigen Schichtsilikaten mit Montmorillonit-Struktur, mit einem Aluminiumgehalt kleiner 0,3 Gew.-%.

Ebenfalls Gegenstand der vorliegenden Erfindung 'ist ein Verfahren zur Verringerung des Aluminiumgehalts eines Katalysatorträgers mit einem Aluminiumgehalt im Katalysatorträger nach der Behandlung von Kleiner 0.3 Gew. %, der im wesentlichen aluminiumhaltige Schichtsilikate mit Montmorillonit-Struktur umfaßt, dadurch gekennzeichnet, daß man den Katalysatorträger
- mit einer Säure tränkt,
- hydrothermal bei einer Temperatur von 160 bis 300 °C und einem Wasserdampfpartialdruck von 4 bis 80 bar_{absolut} behandelt,
- nachfolgend mit saurer, basischer oder neutraler Lösung bei einer Temperatur von 20 bis 100 °C wäscht und
- anschließend mit Wasser bis zur Neutralität des Waschwassers nachwäscht.

Außerdem ist Gegenstand der vorliegenden Erfindung ein Verfahren zur Hydratisierung von C₂- oder C₃-Olefinen mit Wasser in Gegenwart eines Katalysators, der aus einem mit Säure getränkten Katalysatorträger gemäß zumindest einem der Ansprüche 1 bis 24 besteht.

Unter Hydratisierung bzw. Hydratisierungsreaktionen wird im Sinne der vorliegenden Erfindung die Reaktion von Wasser mit einer Kohlenstoff-Kohlenstoff-Doppelbindung verstanden.

Unter Entaluminierung bzw. entaluminierten Katalysatorträgern wird im Sinne der vorliegenden Erfindung ein Verfahren zur Verringerung des Aluminiumgehalts bzw. ein Katalysatorträger mit einem verringertem Aluminiumgehalt verstanden.

Durch Einsatz des erfindungsgemäßen Verfahrens kann ein auf Basis von kalzinierten und nachbehandelten Schichtsilikaten basierender Katalysatorträger hergestellt werden, der einen deutlich geringeren Gehalt an Aluminium aufweist als ein nicht erfindungsgemäß behandelter Katalysatorträger. Trotz des geringeren Aluminiumgehalts behält der Katalysator seine Langzeitstabilität. Durch Einsatz des erfindungsgemäßen Katalysatorträgers in dem erfindungsgemäßen Verfahren zur Hydratisierung von C₂- oder C₃-Olefinen mit Wasser wird der Anteil an während der Hydratisierungsreaktion ausgewaschenem Aluminium deutlich verringert. Dadurch entstehen während der Hydratisierungsreaktion weniger schwer lösliche Aluminiumverbindungen, die bei herkömmlichen Verfahren die Standzeiten nachgeschalteter Apparaturen, wie z.B. Wärmetauscher, verringern, indem sie Leitungen oder Wärmetauscherflächen zusetzen.

Der erfindungsgemäße entaluminierte Katalysatorträger mit einem Aluminiumgehalt kleiner 0,3 Gew.-%, enthält im wesentlichen aluminiumhaltige Schichtsilikate. Besonders bevorzugt hat der erfindungsgemäße entaluminierte Katalysatorträger einen Aluminiumgehalt kleiner 0,03 Gew.-%. Die aluminiumhaltigen Schichtsilikate sind vorzugsweise Smektite und weisen vorzugsweise Montmorillonit-Struktur auf Schichtsilikate, welche im wesentlichen aluminiumhaltige Schichtsilikate mit Montmorillonit-Struktur aufweisen, sind z.B. die Bentonite. Neben den Montmorilloniten können die Bentonite als weitere Bestandteile z.B. Glimmer, Illit, Cristobalit und Zeolithe enthalten.

Ausgangsstoff für die Herstellung des erfindungsgemäßen Katalysatorträgers sind handelsübliche Katalysatorträger z.B. auf Basis von kalzinierten und nachbehandelten Schichtsilikaten.

Der erfindungsgemäße entaluminierte Katalysatorträger mit einem Aluminiumgehalt kleiner 0,3 Gew.-%, vorzugsweise kleiner 0,03 Gew.-%, auf Basis von im wesentlichen aluminiumreichen Schichtsilikaten mit Montmorillonit-Struktur, kann durch Tränken des Katalysatorträgers mit einer Säure, vorzugsweise einer Mineralsäure und besonders bevorzugt einer Phosphorsäure, vorzugsweise einer 10 bis 90 Gew.-%igen Phosphorsäure, besonders bevorzugt einer 50 bis 60 Gew.-%igen Phosphorsäure, so daß der Katalysatorträger 5 bis 60 %, vorzugsweise 30 bis 40 % einer Säure, vorzugsweise einer Phosphorsäure, enthält, darauffolgendes hydrothermales Behandeln bei einer Temperatur von 160 bis 300 °C, vorzugsweise bei einer Temperatur von 220 bis 260 °C und einem Wasserdampfpartialdruck von 4 bis 80 bar_{absolut}, vorzugsweise bei einem Wasserdampfpartialdruck von 16 bis 25 bar_{absolut}, nachfolgender Wäsche mit saurer, basischer oder neutraler, vorzugsweise saurer oder neutraler Lösung, besonders bevorzugt mit Wasser, Salzsäure oder Wasser, welches 0 bis 30 Teile konzentrierte Salzsäure enthält, bei einer Temperatur von 20 bis 100 °C, vorzugsweise von 70 bis 90 °C und anschließendes Nachwaschen des Katalysatorträgers bis zur Neutralität des Waschwassers erhalten werden.

Eine beispielhafte Ausführungsart des erfindungsgemäßen Verfahrens zur Verringerung des Aluminiumgehalts eines Katalysatorträgers wird im folgenden beschrieben, ohne daß das erfindungsgemäße Verfahren auf diese beschränkt ist.

Zur Verringerung des Aluminiumgehalts eines Katalysatorträgers, der im wesentlichen aluminiumhaltige Schichtsilikate umfaßt, können handelsübliche, Schichtsilikate, wie z.B. Montmorillonite oder Bentonite, enthaltende Katalysatorträger verwendet werden. Die Katalysatorträger haben vorzugsweise die Form von sphärischen Körpern, wie z.B. Kugeln, Linsen, Quadern, Zylindern oder auch unregelmäßige Formen, besonders bevorzugt haben sie die Form von Kugeln. Die sphärischen Körper weisen vorzugsweise einen durchschnittlichen Durchmesser von 1 bis 10 mm, besonders bevorzugt einen von 4 bis 6 mm, auf.

Zur Verringerung des Aluminiumgehaltes im Katalysatorträger wird der Katalysatorträger in Säure getränkt, hydrothermal behandelt, anschließend gewaschen und schließlich nachgewaschen.

Der Katalysatorträger wird zum Erzielen des erfindungsgemäßen Effekts in Säure, vorzugsweise in einer Mineralsäure und ganz besonders bevorzugt in Phosphorsäure, getränkt. Es wird eine 10 bis 90 Gew.-%ige Phosphorsäure, vorzugsweise eine 50 bis 60 Gew.-%ige Phosphorsäure verwendet. Nach dem Tränken sollte der Katalysatorträger einen Gehalt an Säure, vorzugsweise Phosphorsäure, von 5 bis 60 Gew.-%, vorzugsweise 30 bis 40 Gew.-%, aufweisen. Der Katalysatorträger wird anschließend hydrothermal behandelt.

Bei den hydrothermalen Bedingungen wandeln sich Schichtsilikatmaterialien, wie z.B. Montmorillonit in cristobalitähnliche Strukturen um. Einhergehend verschwinden die ursprünglich vorhandenen Mikroporen. Diese morphologischen Strukturveränderungen zeigen sich deutlich in der BET-Oberfläche, dem Porenvolumen und der Porenradienverteilung. Unter den hydrothermalen Reaktionsbedingungen gelangt man zu sogenannten "offenen" Porenstrükturen.

Die hydrothermale Behandlung des schichtsilikathaltigen Katalysatorträgers kann bei Temperaturen zwischen 160 und 300 °C und einem Wasserdampfpartialdruck zwischen 4 und 80 bar_{absolut}, bevorzugt zwischen 220 und 260 °C und einem Wasserdampfpartialdruck von 16 bis 25 bar_{absolut}, erfolgen.

Nach der hydrothermalen Behandlung wird der Katalysatorträger mit einer basischen, sauren oder neutralen Lösung, vorzugsweise mit einer sauren oder neutralen Lösung, besonders bevorzugt mit Salzsäure, mit Wasser, welches 0 bis 30 Teile konzentrierte Salzsäure enthält oder mit einer neutralen wäßrigen Lösung, gewaschen. Das Waschen des Katalysatorträgers wird bei einer Temperatur von 20 bis 100 °C, vorzugsweise von 70 bis 90 °C, durchgeführt.

Nach dem Waschen kann der Katalysatorträger mit Wasser bis zur Neutralität des Waschwassers nachgewaschen werden.

Die Katalysatorträger weisen ein Gesamtporenvolumen von 0,2 bis 0,9 ml/g, besonders bevorzugt zwischen 0,6 und 0,7 ml/g, auf. Die Druckfestigkeit der Katalysatorträger sollte zumindest 10 N/mm, vorzugsweise zumindest 20 N/mm betragen.

In einer besonderen Ausführungsart des erfindungsgemäßen Verfahren erfolgt die hydrothermale Behandlung des mit Säure getränkten Katalysatorträgers, der 5 bis 60 Gew.-%, vorzugsweise 30 bis 40 Gew.-%, Phosphorsäure enthält, durch Einsatz als Katalysator in einer Hydratisierungsreaktion von C₂- oder C₃-Olefinen. Zum Tränken des Katalysatorträgers wird vorzugsweise eine 10 bis 90 Gew.-%ige, besonders bevorzugt eine 50 bis 60 Gew.-%ige, Phosphorsäure verwendet.

Bei dieser Hydratisierungsreaktion wird in einem mit dem Katalysator gefüllten Reaktor, vorzugsweise einem Rohrreaktor, Olefin und Wasser in einem Molverhältnis von 0,1 bis 0,8, vorzugsweise von 0,15 und 0,5 zur Reaktion gebracht. Das eingesetzte Olefin und das eingesetzte Wasser werden gasförmig oder flüssig, vorzugsweise gasförmig, in den Reaktor gefahren. Zum Verdampfen des Wassers bzw. zum Heizen beider Edukte auf Reaktionstemperatur kann es vorteilhaft sein, beide Edukte über eine Verdampfer- und/oder Thermostatisierungsstrecke, die elektrisch oder mittels Wärmeträger auf Reaktionstemperatur beheizt sind, in den Reaktor zu führen. Die Gas-Hourly-Space-Velocity (GHSV) sollte zwischen 10 und 100 lₙ/min/l_{Kat} betragen. Die Hydratisierungsreaktion von wird bei einer Temperatur von 160 bis 300 °C und einem Druck von 20 bis 200 bar_{absolut} ausgeführt. Die Hydratisierung von Ethen zu Ethanol wird vorzugsweise bei einer Temperatur von 220 bis 260 °C und einem Druck von 60 bis 80 bar_{absolut} durchgeführt.

Der Reaktorausgang kann vorzugsweise mit einem Kühler verbunden sein, der einen Großteil der unterkritischen Komponenten auskondensiert und diese weiteren Aufarbeitungsschritten, z.B. einer destillativen Trennung, zugänglich macht.

Zur Kontrolle der Aktivität und Selektivität des mit Säure getränkten Katalysatorträgers kann es vorteilhaft sein, den Austrittsstrom aus dem Reaktor zu analysieren. Die Analyse kann z.B. gaschromatographisch erfolgen.

Zur Verlängerung der Standzeit des Katalysators kann es vorteilhaft sein kontinuierlich oder diskontinuierlich, vorzugsweise kontinuierlich die Säure mit der der Katalysatorträger getränkt wurde, in den Reaktor nachzudosieren. Das Einbringen der Säure in den Reaktor kann z.B. durch Eindüsen erfolgen. Die Menge der Säure, die in den Reaktor eingebracht wird, kann von dem Analyseergebnis des Austrittsstroms abhängig gemacht werden. Die Analyse des Austrittstroms und die Bestimmung der daraus resultierenden Säuremenge, die zugegeben wird, kann automatisch erfolgen.

Nach der hydrothermalen Behandlung des Katalysatorträgers, durch Einsatz als Katalysator in einer Hydratisierungsreaktion, wird die Restsäure, mit welcher der Katalysatorträger getränkt wurde, durch Waschen mit Wasser bis zur Neutralität des Waschwassers entfernt.

Nach dem Entfernen der Restsäure wird der Katalysatorträger mit einer basischen, sauren oder neutralen Lösung, vorzugsweise mit einer sauren oder neutralen Lösung, besonders bevorzugt mit Salzsäure, mit Wasser, welches 0 bis 30 Teile konzentrierte Salzsäure enthält oder mit einer neutralen wäßrigen Lösung, gewaschen. Der Katalysatorträger kann bei einer Temperatur von 20 bis 100 °C, vorzugsweise bei einer Temperatur von 70 bis 90 °C gewaschen werden.

Nach dem Waschen kann der Katalysatorträger mit Wasser bis zur Neutralität des Waschwassers nachgewaschen werden.

Bei Katalysatorträgern die durch Verwendung als Katalysator in einer Hydratisierungsreaktion hydrothermal behandelt wurden kann es vorteilhaft sein, nach dem Verringern des Aluminiumgehaltes im Katalysatorträger, den Katalysatorträger durch Abbrennen eventuell anhaftender kohlenstoffhaltiger Verbindungen bei 300 bis 1 000 °C, vorzugsweise bei 450 bis 500 °C, zu reinigen.

Bei beiden Ausführungsarten des erfindungsgemäßen Verfahrens erhält man einen behandelten Katalysatorträger mit einem verringerten Gehalt an Aluminium. Die behandelten Katalysatorträger haben einen durchschnittlichen Durchmesser von 1 bis 10 mm, bevorzugt einen von 4 bis 6 mm. Das Gesamtporenvolumen beträgt von 0,2 bis 0,9 ml/g, vorzugsweise von 0,6 bis 0,7 ml/g. Die Druckfestigkeit nach der Behandlung des Katalysatorträgers beträgt zumindest 10 N/mm, vorzugsweise zunmindest 20 N/mm. Der Aluminiumgehalt der behandelten Katalysatorträger ist kleiner 0,3 Gew.-%, vorzugsweise kleiner 0,03 Gew.-%.

Die nach dem erfindungsgemäßen Verfahren hergestellten Katalysatorträger mit verringertem Aluminiumgehalt können zur Herstellung von Katalysatoren verwendet werden.

Die nach dem erfindungsgemäßen Verfahren hergestellten Katalysatorträger mit verringertem Aluminiumgehalt können erfindungsgemäß zur Hydratisierung von C₂- oder C₃-Olefinen mit Wasser in Gegenwart eines Katalysators, der aus einem mit Säure getränkten, erfindungsgemäß behandelten Katalysatorträger besteht, eingesetzt werden.

Vorzugsweise wird der Katalysatorträger mit einer Säure, vorzugsweise Phosphorsäure, getränkt. Der Phosphorsäuregehalt des getränkten Katalysatorträgers sollte zum Erreichen einer maximalen Katalysatoraktivität zwischen 5 bis 60 Gew.-% liegen, bevorzugt zwischen 30 bis 40 Gew.-%. Zum Tränken des Katalysatorträgers wird eine wäßrige Phosphorsäurelösung mit einem Phosphorsäuregehalt von 10 bis 90 Gew.-%, vorzugsweise von 50 bis 60 Gew.-% eingesetzt. Der so hergestellte saure Katalysator wird in einen Reaktor, vorzugsweise einen Rohrreaktor, gefüllt. Der Reaktor wird isotherm oder nicht isotherm, vorzugsweise isotherm, betrieben und kann elektrisch oder mittels Wärmeträgern beheizt werden.

Der Reaktor wird kontinuierlich oder diskontinuierlich, vorzugsweise kontinuierlich mit den Edukten Wasser und C₂- oder C₃-Olefin beschickt. Das Verhältnis von Wasser zu Olefin mit welchem die Edukte in den Reaktor gefahren werden wird auf ein Molverhältnis von 0,1 bis 0,8, vorzugsweise von 0,15 bis 0,5, eingestellt. Das Einstellen des Molverhältnisses kann z.B. mit Hilfe von Massendurchflußreglern geschehen. Beide Edukte können flüssig oder gasförmig, vorzugsweise gasförmig, in den Reaktor gefahren werden. Zum Verdampfen des Wassers bzw. zum Heizen beider Edukte auf Reaktionstemperatur kann es vorteilhaft sein, beide Edukte über eine Verdampfer- und/oder Thermostatisierstrecke, die elektrisch oder mittels Wärmeträgern auf Reaktionstemperatur beheizt sind, in den Reaktor zu führen. Die Temperatur im Reaktor und die Temperatur, mit welcher die Edukte in den Reaktor strömen, beträgt 160 bis 300 °C. Bei der Hydratisierung von Ethen zu Ethanol beträgt die Temperatur im Reaktor und die Temperatur, mit welcher die Edukte in den Reaktor strömen, vorzugsweise 220 bis 260 °C. Der Druck im Reaktor wird im Bereich von 20 bis 200 bar_{absolut}, vorzugsweise von 60 bis 80 bar_{absolut} eingestellt.

Der Reaktorausgang ist vorzugsweise mit einem Kühler verbunden, der einen Großteil der unterkritischen Komponenten auskondensiert und diese weiteren Aufarbeitungsschritten zuführt.

Zur Kontrolle der Aktivität und Selektivität des mit Säure getränkten Katalysatorträgers kann es vorteilhaft sein, den Austrittsstrom aus dem Reaktor zu analysieren. Die Analyse kann z.B. gaschromatographisch erfolgen.

Zur Verlängerung der Standzeit des Katalysators kann es vorteilhaft sein kontinuierlich oder diskontinuierlich, vorzugsweise kontinuierlich, die Säure, vorzugsweise Phosphorsäure, mit welcher der Katalysatorträger getränkt wurde, in den Reaktor einzubringen. Das Einbringen der Säure in den Reaktor kann z.B. durch Eindüsen erfolgen. Die Menge der Säure, die in den Reaktor eingebracht wird, kann von dem Analyseergebnis des Austrittsstroms abhängig gemacht werden. Die Analyse des Austrittstroms und die Bestimmung der daraus resultierenden Säuremenge die zugegeben wird kann automatisch erfolgen.

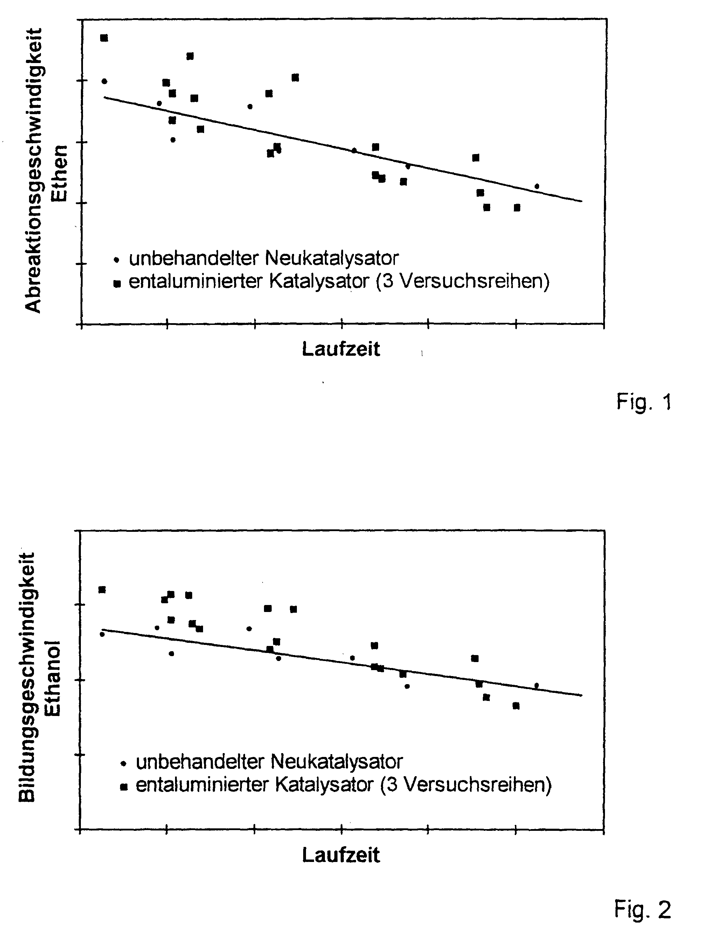

In Figur 1 und 2 sind Abreaktionsgeschwindigkeiten für Ethen sowie Bildungsgeschwindigkeiten für Ethanol bei Verwendung unterschiedlich behandelter Katalysatorträger in Abhängigkeit von der Versuchslaufzeit dargestellt, ohne daß das erfindungsgemäße Verfahren auf diese Ergebnisse beschränkt ist.

### Figur 1

In Fig. 1 ist die Abreaktionsgeschwindigkeit von Ethen bei einer Hydratisierungsreaktion in Abhängigkeit von der Versuchslaufzeit dargestellt. Es sind die Meßwerte von vier Versuchen abgebildet. Die mit Kreisen gekennzeichneten Meßwerte geben die Abreaktionsgeschwindigkeit von Ethen in Bezug auf die Laufzeit wieder, wenn ein Neukatalysatorträger mit dem ursprünglichen Gehalt an Aluminium verwendet wird. Die als Vierecke dargestellten Meßpunkte der Abreaktionsgeschwindigkeit resultieren aus drei Versuchsreihen, die unter Verwendung eines Katalysatorträgers mit verringertem Aluminiumgehalt durchgeführt wurden.

### Figur 2

In Fig. 2 ist die Bildungsgeschwindigkeit von Ethanol bei einer Hydratisierungsreaktion in Abhängigkeit von der Laufzeit der Versuche dargestellt. Es sind die Meßwerte von vier Versuchen abgebildet. Die mit Kreisen gekennzeichneten Meßwerte geben die Bildungsgeschwindigkeit von Ethanol in Bezug auf die Laufzeit wieder, wenn ein Neukatalysatorträger mit dem ursprünglichen Gehalt an Aluminium verwendet wird. Die als Vierecke dargestellten Meßpunkte der Bildungsgeschwindigkeit von Ethanol resultieren aus drei Versuchsreihen, die unter Verwendung eines Katalysatorträgers mit verringertem Aluminiumgehalt durchgeführt wurden.

Das erfindungsgemäße Verfahren wird anhand der folgenden Beispiele beschrieben, ohne darauf beschränkt zu sein.

### Beispiel 1: Ethanolsynthese mit einem unbehandelten Katalysatorträger

Der Versuch erfolgte in einer Pilotanlage, die als Kernstück einen isotherm betriebenen Rohrreaktor von 1 000 mm Länge und 48 mm Durchmesser besitzt.

Die Edukte Wasser und Ethen werden über eine Verdampfer- bzw. Thermostatisierstrecke, die elektrisch auf Reaktionstemperatur beheizt ist, dem Reaktor zugeführt. Das Wasser wird über eine Pumpe flüssig zudosiert, während das Ethen aus 130-bar Stahlflaschen entnommen wird. Der Zustrom eines 0,3 : 1 Gemisches aus Ethen und Wasser (Molbasis) wird über Massendurchflußregler eingestellt.

Der Reaktorausgang ist mit einem Kühler verbunden, um den Großteil der unterkritischen Komponenten - im wesentlichen Ethanol, Wasser und Diethylether - auszukondensieren; der Rest gelangt ins Abgas, dessen Volumenstrom mit einer Gasuhr bestimmbar ist. Ein Teil des Abgases wird über einen Bypass-Strom einem Gaschromatographen zugeführt. Der Flüssigaustrag wird ebenfalls gaschromatographisch analysiert.

Die Synthese von Ethanol wurde im vorliegenden Beispiel bei 240 °C und 70 bar_{abs}. vermessen. Die Standardtestbedingungen sind in Tabelle 1 zusammengefaßt. Als Katalysator wurde ein unbehandelter Neukatalysatorträger, KA-1 der Südchemie AG, eingesetzt. Die Trägerkenndaten sind in Tabelle 2 zusammengestellt. Die erzielten Umsatz- und Selektivitätswerte zu Versuchsbeginn sind ebenfalls in Tabelle 2 enthalten.

Zur Bestimmung des Aluminiumgehaltes des Katalysatorträgers wurde dieser vor der Durchführung des Versuches mit einem Atomemissionsspektrometer analysiert, um den Gehalt an Aluminium zu bestimmen. Als Atomemissionsspektrometer wurde ein induktivgekoppeltes Plasmaatomemissionsspektrometer (ICP-AES) JY 38+ der Firma ISA Jobin Y von verwendet. Die Ergebnisse der Analyse werden in Tabelle 2 wiedergegeben.

### Beispiel 2: Ethanolsynthese mit einem unbehandelten Altträger

Der Versuch wurde wie in Beispiel 1 beschrieben wiederholt. Es wurde diesmal ein schon einmal für die Katalyse einer Hydratisierungssreaktion benutzter unbehandeiter Katalysatorträger (Altträger) als Katalysatorträger verwendet. Wiederum gelten die in Tabelle 1 angegebenen Standard-Testbedingungen. Die Ergebnisse des Versuches sowie die Kenndaten des Katalysatorträgers sind ebenfalls wieder in Tabelle 2 wiedergegeben.

Wie den Werten aus Tabelle 2 entnommen werden kann, verringert sich die spezifische Oberfläche des getränkten Katalysatorträgers nach einmaligen Gebrauch als Katalysator. Ebenfalls wird durch die einmalige Verwendung als Katalysator der Aluminiumgehalt auf ca. 1/4 des ursprünglichen Gehalts an Aluminium reduziert. Die restlichen ¾ des ursprünglichen Gehalts an Aluminium des unbehandelten Neuträgers sind während der Hydratisierungsreaktion aus dem Katalysatorträger herausgewaschen worden. Dieses Aluminium bildet die schwerlöslichen Rückstände, die sich bei anschließenden Aufarbeitungsschritten als hinderlich erwiesen haben.

### Beispiel 3: Ethanolsynthese mit behandeltem Altträger

Der Versuch wurde wie in Beispiel 1 beschrieben wiederholt. Als Katalysatorträger kam ein schon benutzter Altträger zum Einsatz, dessen Gehalt an Aluminium durch Behandlung nach dem erfindungsgemäßen Verfahren verringert wurde. Wiederum gelten die in Tabelle 1 angegebenen Standard-Testbedingungen. Die Ergebnisse des Versuches sowie die Kenndaten des Katalysatorträgers sind ebenfalls wieder in Tabelle 2 wiedergegeben.

Das Desaktivierungsverhalten eines Katalysatorträgers ohne verringertem Aluminiumgehalt und eines mit verringertem Aluminiumgehalt ist in der Fig.1 und Fig.2 dargestellt.

Wie aus Tabelle 2 deutlich wird, ist durch Behandlung des Altträgers mit Hilfe des erfindungsgemäßen Verfahrens der Aluminiumgehalt des Katalysatorträgers auf unter 0,03 Gew.-% reduziert worden. Dieser Wert stellt die Nachweisgrenze des benutzten Atomemissionsspektrometers dar. Die Druckfestigkeit des behandelten Altträgers ist mit 30 N/mm immer noch ausreichend, um eine gute Langzeitstabilität des Katalysatorträgers zu garantieren.

Trotz Behandlung des Katalysatorträgers und Reduzierung des Aluminiumgehaltes auf unter 0,03 Gew.-% ist der Etylenumsatz und die Ausbeute an Ethanol im Vergleich zum unbehandelten und unbenutzten Katalysatorträger (Neuträger) bzw. zum unbehandelten Altträger gleich gut geblieben, im vorliegenden Versuch sogar leicht verbessert worden.

Wie den Figuren 1 und 2 entnommen werden kann, hat die Verringerung des Aluminiumgehaltes nach dem erfindungsgemäßen Verfahren keinen Einfluß auf die Abreaktionsgeschwindigkeit des Ethens und die Bildungsgeschwindigkeit des Ethanols.

**Tabelle 1**

| **Standard-Testbedingungen** | |
|---|---|
| *Prozeßparameter* | *Wert des Prozeßparameters* |
| Gesamtdruck bei der Reaktion | 70 bar_{abs.} |
| Reaktortemperatur (isotherm) | 240°C |
| GHSV | 21,3 l_{N}/min/l_{Kat.} |
| Wasser-zu-Ethylen-Verhältnis | 1,0 : 0,3 mol : mol |
| Trägermaterial | KA-1 (Südchemie) |

**Tabelle 2**

| Eigenschaft (getränkter Träger) | *Neuträger* | *unbehandelten Altträger* | *behandelter Altträger* |
|---|---|---|---|
| Druckfestigkeit | 20 N/mm | 40 N/mm | 30 N/mm |
| spez. Oberfläche (BET) | 20 m²/g | 4 m²/g | 3 m²/g |
| Porenvolumen_{gesamt} | 0,7 ml/g | 0,4 ml/g | 0,4 ml/g |
| Al-Gehalt | 1,3 Gew.-% | 0,31 Gew.-% | < 0,03 Gew.-% |
| Si-Gehalt | 25 Gew.-% | 25 Gew.-% | 24 Gew.-% |
| H₃PO₄-Gehalt | 35 Gew.-% | 36 Gew.-% | 35 Gew.-% |
| Ethylenumsatz zu Versuchsbeginn | 5 % | 5 % | 6 % |
| Raum-Zeit-Ausbeute (Ethanol) zu Versuchsbeginn | 77,4 g/l_{Kat.}/h | 76,4 g/l_{Kat.}/h | 79,8 g/l_{Kat.}/h |

## Patentansprüche

1. Entaluminierter Katalysatorträger, auf Basis von im wesentlichen aluminiumhaltigen Schichtsilikaten mit Montmorillonit-Struktur, mit einem Aluminiumgehalt kleiner 0,3 Gew.-%.

2. Katalysatorträger nach Anspruch 1, der durch Tränken mit Phosphorsäure, darauffolgender hydrothermaler Behandlung bei einer Temperatur von 160 bis 300 °C und einem Wasserdampfpartialdruck von 4 bis 80 bar_{absolut}, nachfolgender Wäsche mit saurer, basischer oder neutraler Lösung bei einer Temperatur von 20 bis 100 °C und anschließendes Nachwaschen mit Wasser bis zur Neutralität des Waschwassers erhältlich ist.

3. Katalysatorträger nach zumindest einem der Ansprüche 1 oder 2, mit einem Aluminiumgehalt kleiner 0,03 Gew.-%.

4. Verfahren zur Verringerung des Aluminiumgehalts eines Katalysatorträgers auf einem Aluminiumgehalt im Katalysatorträger nach der Behandlung von Kleiner 0.3 gew. %, der im wesentlichen aluminiumhaltige Schichtsilikate mit Montmorillonit-Struktur umfaßt,
**dadurch gekennzeichnet, daß** man den Katalysatorträger
- mit einer Säure tränkt,
- hydrothermal bei einer Temperatur von 160 bis 300 °C und einem Wasserdampfpartialdruck von 4 bis 80 bar_{absolut} behandelt,
- nachfolgend mit saurer, basischer oder neutraler Lösung bei einer Temperatur von 20 bis 100 °C wäscht und
- anschließend mit Wasser bis zur Neutralität des Waschwassers nachwäscht.

5. Verfahren nach Anspruch 4,
**dadurch gekennzeichnet,**
**daß** der Katalysatorträger bei einer Temperatur von 220 bis 260 °C und einem Wasserdampfpartialdruck von 16 bis 25 bar_{absolut} hydrothermal behandelt wird.

6. Verfahren nach Anspruch 4,
**dadurch gekennzeichnet,**
**daß** der Katalysatorträger durch einen Einsatz als Katalysator in einer Hydratisierungsreaktion hydrothermal behandelt wird.

7. Verfahren nach Anspruch 6,
**dadurch gekennzeichnet,**
**daß** der Katalysatorträger durch Abbrennen bei 300 bis 1000 °C von anhaftenden organischen kohlenstoffhaltigen Verbindungen gereinigt wird.

8. Verfahren nach zumindest einem der Ansprüche 4 bis 7,
**dadurch gekennzeichnet,**
**daß** der Katalysatorträger zum Waschen mit Wasser behandelt wird.

9. Verfahren nach zumindest einem der Ansprüche 4 bis 8,
**dadurch gekennzeichnet,**
**daß** der Katalysatorträger zum Waschen mit Salzsäure behandelt wird.

10. Verfahren nach zumindest einem der Ansprüche 4 bis 9,
**dadurch gekennzeichnet,**
**daß** der Katalysatorträger zum Waschen mit Wasser, welches 0 bis 30 Teile konzentrierte Salzsäure enthält, behandelt wird.

11. Verfahren nach zumindest einem der Ansprüche 4 bis 10,
**dadurch gekennzeichnet,**
**daß** das Waschen bei einer Temperatur von 70 bis 90 °C erfolgt.

12. Verfahren nach zumindest einem der Ansprüche 4 bis 11,
**dadurch gekennzeichnet,**
**daß** der Katalysatorträger die Form eines sphärischen Körpers aufweist.

13. Verfahren nach Anspruch 12,
**dadurch gekennzeichnet,**
**daß** der Katalysatorträger die Form einer Kugel aufweist.

14. Verfahren nach zumindest einem der Ansprüche 12 oder 13,
**dadurch gekennzeichnet,**
**daß** der Katalysatorträger einen Durchmesser von 1 bis 10 mm aufweist.

15. Verfahren nach Anspruch 14,
**dadurch gekennzeichnet,**
**daß** der Katalysatorträger einen Durchmesser von 4 bis 6 mm aufweist.

16. Verfahren nach zumindest einem der Ansprüche 4 bis 15,
**dadurch gekennzeichnet,**
**daß** der Katalysatorträger ein Gesamtporenvolumen von 0,2 bis 0,9 ml/g aufweist:

17. Verfahren nach Anspruch 16,
**dadurch gekennzeichnet,**
**daß** der Katalysatorträger ein Gesamtporenvolumen von 0,6 bis 0,7 ml/g aufweist.

18. Verfahren nach zumindest einem der Ansprüche 4 bis 17,
**dadurch gekennzeichnet,**
**daß** der Katalysatorträger eine Druckfestigkeit von zumindest 10 N/mm aufweist.

19. Verfahren nach Anspruch 18,
**dadurch gekennzeichnet,**
**daß** der Katalysatorträger eine Druckfestigkeit von zumindest 20 N/mm aufweist.

20. Verfahren nach zumindest einem der Ansprüche 4 bis 19,
**dadurch gekennzeichnet,**
**daß** der Katalysatorträger auf Basis von Montmorillonit hergestellt ist.

21. Verfahren nach Anspruch 4,
**dadurch gekennzeichnet,**
**daß** der Aluminiumgehalt im Katalysatorträger nach der Behandlung kleiner 0,03 Gew.-% ist.

22. Verfahren nach zumindest einem der Ansprüche 4 bis 21,
**dadurch gekennzeichnet,**
**daß** der Katalysatorträger mit einer Mineralsäure getränkt wird.

23. Verfahren nach zumindest einem der Ansprüche 4 bis 22,
**dadurch gekennzeichnet,**
**daß** der Katalysatorträger mit Phosphorsäure getränkt wird.

24. Verfahren zur Hydratisierung von C₂- oder C₃-Olefinen mit Wasser in Gegenwart eines Katalysators, der aus einem mit Säure getränkten Katalysatorträger gemäß einem der Ansprüche 1 bis 3 besteht, oder der nach einem der Ansprüche 4-23 hergestellt wird.

25. Verfahren nach Anspruch 24, **dadurch gekennzeichnet, daß**
- die Hydratisierungsreaktion in einem Reaktor durchgeführt wird,
- das Molverhältnis von Olefin zu Wasser im Reaktor 0,1 bis 0,8 beträgt,
- die Gas-Hourly-Space-Velocity von 10 bis 100 lₙ/min/l_{Kat} beträgt,
- der Katalysator 5 bis 60 Gew.-% Säure enthält und
- die Hydratisierungsreaktion der Olefine bei einer Temperatur von 170 bis 300 °C sowie bei einem Druck von 20 bis 200 bar_{absolut} durchgeführt wird.

26. Verfahren nach zumindest einem der Ansprüche 24 und 25,
**dadurch gekennzeichnet,**
**daß** die Säure, mit welcher der Katalysatorträger getränkt wird, eine 10 bis 90 Gew.-%ige Phosphorsäure ist.

27. Verfahren nach Anspruch 26,
**dadurch gekennzeichnet,**
**daß** die Säure, mit welcher der Katalysatorträger getränkt wird, eine 50 bis 60 Gew.-%ige Phosphorsäure ist.

28. Verfahren nach zumindest einem der Ansprüche 24 bis 27,
**dadurch gekennzeichnet,**
**daß** der Katalysator 30 bis 40 Gew.-% Phosphorsäure enthält.

29. Verfahren nach zumindest einem der Ansprüche 24 bis 28,
**dadurch gekennzeichnet,**
**daß** die Hydratisierungsreaktion für die Herstellung von Ethanol aus Ethen bei Temperaturen von 220 bis 260 °C und bei einem Druck von 60 bis 80 bar ausgeführt wird.

30. Verfahren nach zumindest einem der Ansprüche 24 bis 29,
**dadurch gekennzeichnet,**
**daß** das eingesetzte Olefin und das eingesetzte Wasser gasförmig in den Reaktor gefahren werden.

31. Verfahren nach zumindest einem der Ansprüche 24 bis 30,
**dadurch gekennzeichnet,**
**daß** während der Hydratisierungsreaktion Säure in den Reaktor eingebracht wird.

32. Verfahren nach Anspruch 31,
**dadurch gekennzeichnet,**
**daß** als Säure Phosphorsäure verwendet wird.

33. Verfahren nach zumindest einem der Ansprüche 31 oder 32,
**dadurch gekennzeichnet,**
**daß** die Säure in den Reaktor kontinuierlich eingedüst wird.

## Claims

1. Dealuminised catalyst support, based on substantially aluminium-containing phyllosilicates of montmorillonite structure, with an aluminium content of less than 0.3 wt.%.

2. Catalyst support according to claim 1, which may be obtained by impregnation with phosphoric acid, subsequent hydrothermal treatment at a temperature of from 160 to 300 °C and a partial pressure of water vapour of from 4 to 80 bar_{abs}, subsequent washing with acidic, basic or neutral solution at a temperature of from 20 to 100 °C and subsequent rewashing with water until the washing water is neutral.

3. Catalyst support according to at least one of claims 1 or 2, having an aluminium content of less than 0.03 wt.%.

4. Process for reducing the aluminium content of a catalyst support with an aluminium content in the catalyst support of less than 0.3 wt.% after treatment, comprising substantially aluminium-containing phyllosilicates of montmorillonite structure, **characterised in that** the catalyst support is
- impregnated with acid,
- treated hydrothermally at a temperature of from 160 to 300 °C and a partial pressure of water vapour of from 4 to 80 bar_{abs},
- then washed with acidic, basic or neutral solution at a temperature of from 20 to 100 °C and
- then rewashed with water until the washing water is neutral.

5. Process according to claim 4,
**characterised in that**
the catalyst support is treated hydrothermally at a temperature of from 220 to 260 °C and a partial pressure of water vapour of from 16 to 25 bar_{abs}.

6. Process according to claim 4,
**characterised in that**
the catalyst support is treated hydrothermally through use as a catalyst in a hydration reaction.

7. Process according to claim 6,
**characterised in that**
the catalyst support is cleaned by burning off adherent organic carbon-containing compounds at 300 to 1000 °C.

8. Process according to at least one of claims 4 to 7,
**characterised in that**
the catalyst support is washed with water.

9. Process according to at least one of claims 4 to 8,
**characterised in that**
the catalyst support is washed with hydrochloric acid.

10. Process according to at least one of claims 4 to 9,
**characterised in that**
the catalyst support is washed with water containing from 0 to 30 parts concentrated hydrochloric acid.

11. Process according to at least one of claims 4 to 10,
**characterised in that**
washing is performed at a temperature of from 70 to 90 °C.

12. Process according to at least one of claims 4 to 11,
**characterised in that**
the catalyst support takes the form of a spherical body.

13. Process according to claim 12,
**characterised in that**
the catalyst support takes the form of a ball.

14. Process according to at least one of claims 12 or 13,
**characterised in that**
the catalyst support has a diameter of from 1 to 10 mm.

15. Process according to claim 14,
**characterised in that**
the catalyst support has a diameter of from 4 to 6 mm.

16. Process according to at least one of claims 4 to 15,
**characterised in that**
the catalyst support comprises a total pore volume of from 0.2 to 0.9 ml/g.

17. Process according to claim 16,
**characterised in that**
the catalyst support comprises a total pore volume of from 0.6 to 0.7 ml/g.

18. Process according to at least one of claims 4 to 17,
**characterised in that**
the catalyst support exhibits a compressive strength of at least 10 N/mm.

19. Process according to claim 18,
**characterised in that**
the catalyst support exhibits a compressive strength of at least 20 N/mm.

20. Process according to at least one of claims 4 to 19,
**characterised in that**
the catalyst support is made on the basis of montmorillonite.

21. Process according to claim 4,
**characterised in that**
the aluminium content in the catalyst support is less than 0.03 wt.% after treatment.

22. Process according to at least one of claims 4 to 21,
**characterised in that**
the catalyst support is impregnated with a mineral acid.

23. Process according to at least one of claims 4 to 22,
**characterised in that**
the catalyst support is impregnated with phosphoric acid.

24. Process for hydrating C₂ or C₃ olefins with water in the presence of a catalyst comprising an acid-impregnated catalyst support according to one of claims 1 to 3, or produced according to one of claims 4 to 23.

25. Process according to claim 24, **characterised in that**
- the hydration reaction is performed in a reactor,
- the molar ratio of olefin to water in the reactor is from 0.1 to 0.8,
- the gas hourly space velocity is from 10 to 100 lₙ/min/l_{Cat},
- the catalyst contains from 5 to 60 wt.% acid and
- the hydration reaction of the olefins proceeds at a temperature of from 170 to 300 °C and at a pressure of from 20 to 200 bar_{abs}.

26. Process according to at least one of claims 24 and 25,
**characterised in that**
the acid with which the catalyst support is impregnated is a 10 to 90 wt.% phosphoric acid.

27. Process according to claim 26,
**characterised in that**
the acid with which the catalyst support is impregnated is a 50 to 60 wt.% phosphoric acid.

28. Process according to at least one of claims 24 to 27,
**characterised in that**
the catalyst contains from 30 to 40 wt.% phosphoric acid.

29. Process according to at least one of claims 24 to 28,
**characterised in that**
the hydration reaction for producing ethanol from ethene is performed at temperatures of from 220 to 260 °C and at a pressure of from 60 to 80 bar.

30. Process according to at least one of claims 24 to 29,
**characterised in that**
the olefin used and the water used are introduced into the reactor in gaseous form.

31. Process according to at least one of claims 24 to 30,
**characterised in that**
acid is introduced into the reactor during the hydration reaction.

32. Process according to claim 31,
**characterised in that** phosphoric acid is used as the acid.

33. Process according to at least one of claims 31 or 32,
**characterised in that**
the acid is injected continuously into the reactor.

## Revendications

1. Support de catalyseur désaluminé, à base de silicates stratifiés contenant essentiellement de l'aluminium, d'une structure de montmorillonite, d'une teneur en aluminium inférieure à 0,3 % en poids.

2. Support de catalyseur selon la revendication 1, qui peut être obtenu par imprégnation avec de l'acide phosphorique, suivie d'un traitement hydrothermal à une température de 160 à 300°C et sous une pression partielle de vapeur d'eau de 4 à 80 bars absolus, puis d'un lavage avec une solution acide, basique ou neutre, à une température de 20 à 100°C et d'un lavage supplémentaire à l'eau jusqu'à neutralité de l'eau de lavage.

3. Support de catalyseur selon au moins l'une des revendications 1 ou 2, présentant une teneur en aluminium inférieure à 0,03 % en poids.

4. Procédé pour diminuer la teneur en aluminium d'un support de catalyseur à une teneur en aluminium dans le support de catalyseur après le traitement de moins de 0,3 % en poids, qui contient des silicates stratifiés essentiellement contenant de l'aluminium, possédant une structure de montmorillonite,
**caractérisé en ce qu'**
• on imprègne le support de catalyseur avec un acide,
• on lui fait subir un traitement hydrothermal à une température de 160 à 300°C et sous une pression partielle de vapeur d'eau de 4 à 80 bars absolus,
• on le lave ensuite avec une solution acide, basique ou neutre, à une température entre 20 et 100°C, et
• on le lave enfin avec de l'eau jusqu'à neutralité de l'eau de lavage.

5. Procédé selon la revendication 4,
**caractérisé en ce que**
le support de catalyseur est soumis à un traitement hydrothermal à une température de 220 à 260°C et sous une pression partielle de vapeur d'eau de 16 à 25 bars absolus.

6. Procédé selon la revendication 4,
**caractérisé en ce que**
le support de catalyseur est soumis à un traitement hydrothermal par une utilisation comme catalyseur dans une réaction d'hydratation.

7. Procédé selon la revendication 6,
**caractérisé en ce que**
le support de catalyseur est purifié par brûlage à 300 à 1000°C des composés carbonés organiques adhérant à ce dernier.

8. Procédé selon au moins l'une des revendications 4 à 7,
**caractérisé en ce que**
le support de catalyseur est traité pour un lavage avec de l'eau.

9. Procédé selon au moins l'une des revendications 4 à 8,
**caractérisé en ce que**
le support de catalyseur est traité pour un lavage avec de l'acide chlorhydrique.

10. Procédé selon au moins l'une des revendications 4 à 9,
**caractérisé en ce que**
le support de catalyseur est traité pour un lavage avec de l'eau qui contient 0 à 30 partie(s) d'acide chlorhydrique concentré.

11. Procédé selon au moins l'une des revendications 4 à 10,
**caractérisé en ce que**
le lavage a lieu à une température de 70 à 90°C.

12. Procédé selon au moins l'une des revendications 4 à 11
**caractérisé en ce que**
le support de catalyseur possède la forme d'un corps sphérique.

13. Procédé selon la revendication 12,
**caractérisé en ce que**
le support de catalyseur a la forme d'une sphère.

14. Procédé selon l'une des revendications 12 ou 13,
**caractérisé en ce que**
le support de catalyseur a un diamètre de 1 à 10 mm.

15. Procédé selon la revendications 14,
**caractérisé en ce que**
le support de catalyseur a un diamètre de 4 à 6 mm.

16. Procédé selon au moins l'une des revendications 4 à 15,
**caractérisé en ce que**
le support de catalyseur possède un volume poreux total de 0,2 à 0,9 ml/g.

17. Procédé selon la revendication 16,
**caractérisé en ce que**
le support de catalyseur possède un volume poreux total de 0,6 à 0,7 ml/g.

18. Procédé selon au moins l'une des revendications 4 à 17,
**caractérisé en ce que**
la support de catalyseur présente une résistance à la compression d'au moins 10 N/mm.

19. Procédé selon la revendication 18,
**caractérisé en ce que**
le support de catalyseur présente une résistance à la compression d'au moins 20 N/mm.

20. Procédé selon au moins une des revendications 4 à 19,
**caractérisé en ce que**
le support de catalyseur est fabriqué à base de montmorillonite.

21. Procédé selon la revendication 4,
**caractérisé en ce que**
la teneur en aluminium dans le support de catalyseur, après traitement, est inférieure à 0,03 % en poids.

22. Procédé selon au moins une des revendications 4 à 21,
**caractérisé en ce que**
le support de catalyseur est imprégné avec un acide minéral.

23. Procédé selon au moins une des revendications 4 à 22,
**caractérisé en ce que**
le support de catalyseur est imprégné avec de l'acide phosphorique.

24. Procédé pour l'hydratation d'oléfines en C₂ ou C₃ avec de l'eau en présence d'un catalyseur qui est constitué d'un support de catalyseur imprégné avec un acide selon l'une quelconque des revendications 1 à 23, ou qui est fabriqué selon l'une quelconque des revendications 4 à 23.

25. Procédé selon la revendication 24,
**caractérisé en ce que**
• la réaction d'hydratation est effectuée dans un réacteur,
• le rapport molaire de l'oléfine à l'eau dans le réacteur est de 0,1 à 0,8,
• la vitesse spatiale horaire à l'état gazeux est de 10 à 100 Iₙ/min/l_{cat},
• le catalyseur contient 5 à 60 % en poids d'acide, et
• la réaction d'hydratation des oléfines est effectuée à une température de 170 à 300°C et sous une pression de 20 à 200 bars absolus.

26. Procédé selon au moins une des revendications 24 et 25,
**caractérisé en ce que**
l'acide avec lequel le support de catalyseur est imprégné est un acide phosphorique à 10 à 90 % en poids.

27. Procédé selon la revendication 26,
**caractérisé en ce que**
l'acide avec lequel le support de catalyseur est imprégné est un acide phosphorique à 50 à 60 % en poids.

28. Procédé selon au moins une des revendications 24 à 27,
**caractérisé en ce que**
le catalyseur contient 30 à 40 % en poids d'acide phosphorique.

29. Procédé selon au moins l'une des revendications 24 à 28,
**caractérisé en ce que**
la réaction d'hydratation pour la fabrication d'éthanol à partir d'éthène est effectuée à des températures de 220 à 260°C et sous une pression de 60 à 80 bars.

30. Procédé selon au moins l'une des revendications 24 à 29,
**caractérisé en ce que**
l'oléfine utilisée et l'eau utilisée sont introduites sous forme gazeuse dans le réacteur.

31. Procédé selon au moins l'une des revendications 24 à 30
**caractérisé en ce que**
de l'acide est introduit dans le réacteur pendant la réaction d'hydratation.

32. Procédé selon la revendication 31,
**caractérisé en ce qu'**
on utilise, comme acide, l'acide phosphorique.

33. Procédé selon au moins une des revendications 31 ou 32,
**caractérisé en ce que**
l'acide est injecté en continu dans le réacteur.
